# EUROPEAN PATENT APPLICATION

(11) **EP 0 845 250 A2**
(43) Date of publication of application: **03.06.1998**
(21) Application number: 97309604.3
(22) Date of filing: 28.11.1997
(51) Int. Cl.: A61F 2/36, A61F 2/40

(54) **An osteoprosthesis component**

(30) Priority: 30.11.1996 GB 9624992
(71) Applicant: DEPUY INTERNATIONAL LIMITED, Beeston, Leeds LS11 8DT (GB)
(72) Inventor: Trail, Iain, Wrightington, Wigan WN6 9EP (GB)
(74) Representative: Belcher, Simon James

(57) **Abstract**

An osteoprosthesis component comprises a head portion with a generally spherical head having a smooth articulating surface, to be received in a socket to form an articulating joint, and a stem portion to be received in the medullary canal of a bone, with an obtuse angle between the axes of the stem portion and the head portion. A part of the articulating surface of the head is provided by a segment of a sphere defined by a plane substantially perpendicular to the axis of the head portion between the articulating surface and the equator of the sphere, the articulating surface of the head including a lip which extends beyond the segment-defining plane, the lip being arranged on that part of the circumference of the head portion facing generally away from the stem portion.

## Description

This invention relates to an osteoprosthesis component, particularly of the type which is used in a ball and socket type joint such as a hip or shoulder.

Components for replacement ball and socket joints comprise a stem component with an elongate stem portion which can be received in the medullary canal of a bone and a head portion. The head of the stem component is received in a socket in which it can articulate. The socket can be provided by natural tissue or by a prosthetic component which provides a cup in which the head portion of the stem component can fit. Articulation of the joint involves relative movement between the socket and the head portion of the stem component received within it.

The range of movement of a replacement joint of this kind depends on the configurations of the stem and socket. The present invention provides a stem component in which the articulating surface of the head portion includes a lip which extends beyond the spherical portion of the said surface, on that part of the circumference of the head portion facing generally away from the stem portion.

Accordingly, in one aspect, the invention provides an osteoprosthesis component which comprises:
(a) a head portion with a generally spherical head having a smooth articulating surface, to be received in a socket to form an articulating joint,
(b) a stem portion to be received in the medullary canal of a bone, with an obtuse angle between the axes of the stem portion and the head portion,
a part of the articulating surface of the head being provided by a segment of a sphere defined by a plane substantially perpendicular to the axis of the head portion between the articulating surface and the equator of the sphere, the articulating surface of the head including a lip which extends beyond the segment-defining plane, the lip being arranged on that part of the circumference of the head portion facing generally away from the stem portion.

The prosthesis of the invention has the advantage that it can permit a range of movement between the stem portion and the socket that is greater than that which is possible when the articulating surface of the head portion of the prosthesis does not extend beyond the plane extending substantially perpendicular to the axis of the head portion. This can allow the range of movement available in a replacement joint using the prosthesis of the invention to be at least as great as that in a natural joint without the stem portion becoming dislocated from the socket. Indeed, the range of movement can be arranged to be greater than that in a natural joint, which can be an advantage when soft tissue components of the joint (such as ligaments) are damaged or otherwise compromised.

Preferably, the lip extends beyond the segment-defining plane through an angle of at least about 20° around the circumference of the component, more preferably at least about 30°, especially at least about 45°. Preferably, the lip extends beyond the plane which defines the spherical portion of the articulating surface of the head through an angle of not more than about 90° around the circumference of the component, more preferably not more than about 75°, especially not more than about 60°.

The lip is arranged on that part of the circumference of the head portion that faces generally away from the stem portion. For example, if the stem portion is considered as extending generally downwardly from the head portion, the lip will be located at or towards at the top of the head portion. Preferably, the point at which the lip of the articulating surface of the head extends furthest from the segment-defining plane lies in the plane in which the axes of the head and stem portions lie. This has been found to provide particular benefit in terms of enhanced freedom of movement of a joint.

Generally, the lip of the articulating surface of the head will be substantially symmetrical about the plane of the axes of the head and stem portions.

Preferably, the distance D from the centre of the sphere to the segment-defining plane is related to the radius R of the sphere by the expression D = NR, where N has a value upto about 1.2. Preferably, the value of N is at least about 0.3, more preferably at least about 0.4, especially at least about 0.5. Preferably, the value of N s less than about 1.1, more preferably less than about 0.9, especially less than about 0.8.

The lip of the articulating surface can form part of the same sphere as the part thereof defined by the segment-defining plane so that substantially all of the articulating surface of the head falls on a sphere. For some applications, it can be appropriate for the curved surface of the lip to have a different shape from that segment portion of the head. For example, the articulating surface on the lip can substantially spherical but with a different radius of curvature, or it might be other than spherical.

Preferably, the lip extends beyond the segment-defining plane through an angle of arc of at least about 15°, more preferably at least about 25°, especially at least about 35°. Preferably, the lip extends beyond the segment-defining plane through an angle of arc of not more than about 50°, more preferably not more than about 40°.

Preferably, the component of the invention is formed of a plurality of modular parts from which the prosthesis can be constructed. This enables a prosthesis to be provided for a particular application in which (a) the stem portion is appropriately configured to fit in the medullary canal, and (b) the head portion is appropriately configured to fit into a socket and to provide the required freedom of movement. For example, the head with its articulating surface can be arranged to be separated from the remainder of the prosthesis, the joined stem and head portions providing a formation to enable the head to be connected, for example by means of a Morse taper.

The materials from which the osteoprosthesis of the invention can be made include materials conventionally used for osteoprostheses. The surface finish requirements of the articulating surface can be achieved using conventional finishing and polishing equipment.

The osteoprosthesis of the invention can be used in ball and socket joints such as hip joints and shoulder joints. It is particularly suitable for use in artificial shoulder joints.

In another aspect, the invention provides a kit of parts for making an osteoprosthesis of the type discussed above, comprising separable head and stem portions which can be connected to one another to form the prosthesis.

In a further aspect, the invention provides a joint prosthesis which comprises an osteoprosthesis of the type discussed above and a cup component in which the head portion of the osteoprosthesis can be received.

The invention is applicable to ball and socket joints generally, with the degree of congruence between the ball and socket (whether prosthetic or natural) components selected according to the requirements of a particular application.

The present invention will now be described, by way of example only, with reference to the accompanying drawings, in which:
Figure 1 is a side view, partially in section, of an osteoprosthesis of the invention,
Figure 2 is a view from below of the head portion of the osteoprosthesis shown in Figure 1, along the axis of the head portion and the arrow "B".

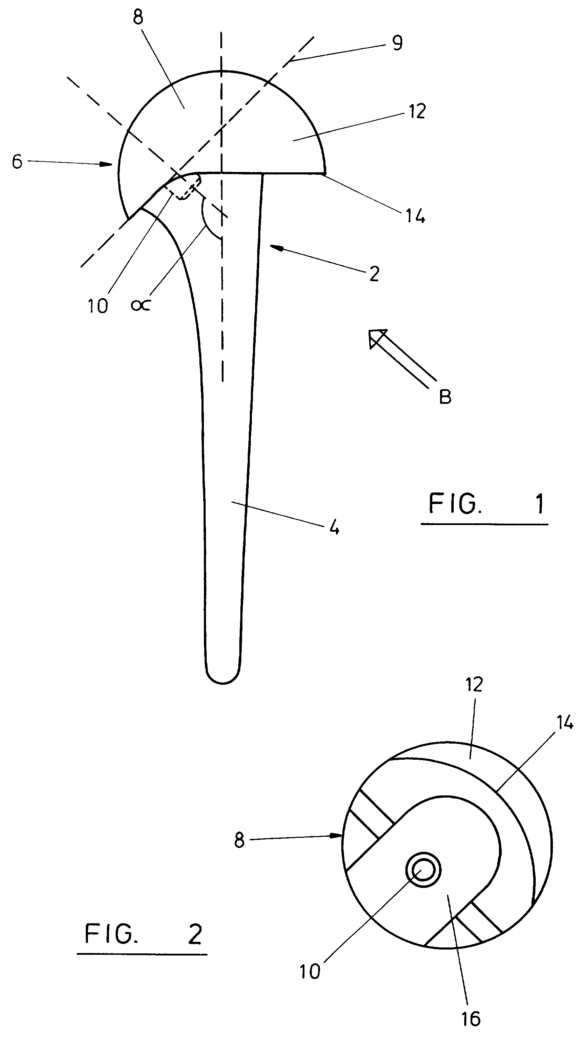

Referring to the drawings, Figures 1 and 2 show an osteoprosthesis 2 for use in a replacement joint of the ball and socket type (such as an artificial hip or shoulder joint). The osteoprosthesis comprises a stem portion 4 which can be received in the medullary canal of a bone. The stem portion can have an appropriately configured surface to facilitate formation of a secure connection to the bone. A head portion 6 extends from the stem portion 4 with an obtuse angle (α) between the axes of the head and stem portions. That angle is about 135°. The head 8 is provided on the head portion. The head can be provided as a separate component, with a tapered recess in it for receiving a correspondingly tapered plug 10 on the head portion.

The head 8 has a generally spherical surface which can be received in a socket to form an articulating joint. The generally spherical surface is defined by a plane 9 substantially perpendicular to the axis of the head portion between the articulating surface and the equator of the sphere and is that provided by a sector of the sphere.

The articulating surface of the head includes a lip portion 12 which extends beyond the sector-defining plane of the head portion, on that part of the circumference of the head portion facing generally away from the stem portion. The point 14 at which the lip of the articulating surface of the head extends furthest from the segment-defining plane lies in the plane in which the axes of the head and stem portions lie. The lip 12 extends beyond the segment-defining plane through an angle of about 170° around the circumference of the component (as can be seen in Figure 2). The lip extends beyond the segment-defining plane through an angle of arc of about 45° (as can be seen in Figure 1).

A recess 16 is provided in the underside of the lip in which the stem portion 4 can be received, to make the connection between the stem and head portions.

## Claims

1. An osteoprosthesis component which comprises:
(a) a head portion with a generally spherical head having a smooth articulating surface, to be received in a socket to form an articulating joint,
(b) a stem portion to be received in the medullary canal of a bone, with an obtuse angle between the axes of the stem portion and the head portion,
a part of the articulating surface of the head being provided by a segment of a sphere defined by a plane substantially perpendicular to the axis of the head portion between the articulating surface and the equator of the sphere, the articulating surface of the head including a lip which extends beyond the segment-defining plane, the lip being arranged on that part of the circumference of the head portion facing generally away from the stem portion.

2. An osteoprosthesis component as claimed in claim 1, in which the lip extends beyond the segment-defining plane through an angle of at least about 20° around the circumference of the component.

3. An osteoprosthesis component as claimed in claim 1 or claim 2, in which the lip extends beyond the segment-defining plane through an angle of not more than about 90° around the circumference of the component.

4. An osteoprosthesis component as claimed in any one of claims 1 to 3, in which the point at which the lip of the articulating surface of the head extends furthest from the segment-defining plane lies in the plane in which the axes of the head and stem portions lie.

5. An osteoprosthesis component as claimed in claim 4, in which the lip of the articulating surface of the head is substantially symmetrical about the plane of the axes of the head and stem portions.

6. An osteoprosthesis component as claimed in any one of claims 1 to 5, in which the distance D from the centre of the sphere to the segment-defining plane is related to the radius R of the sphere by the expression D = NR, where N has a value upto about 1.2.

7. An osteoprosthesis component as claimed in any one of claims 1 to 7, in which the lip of the articulating surface forms part of the same sphere as the segment.

8. An osteoprosthesis component as claimed in claim 7, in which the lip extends beyond the segment-defining plane through an angle of arc of at least about 25°.

9. A joint prosthesis which comprises an osteoprosthesis as claimed in any one of claims 1 to 8, and a cup component in which the head portion of the osteoprosthesis can be received.
